(19) Europäisches Patentamt
European Patent Office
Office européen des brevets

(11) Veröffentlichungsnummer: **0 228 625 B1**

(12) **EUROPÄISCHE PATENTSCHRIFT**

(45) Veröffentlichungstag der Patentschrift: **02.01.92**

(51) Int. Cl.5: **C07K 5/08**, A61K 37/02, A61K 37/64

(21) Anmeldenummer: **86117168.4**

(22) Anmeldetag: **09.12.86**

(54) **Peptid-Derivate mit inhibitorischer Wirkung auf hydroxylierende Enzyme, Verfahren zu ihrer Herstellung, diese enthaltende Mittel und ihre Verwendung.**

(30) Priorität: **14.12.85 DE 3544338**

(43) Veröffentlichungstag der Anmeldung:
**15.07.87 Patentblatt 87/29**

(45) Bekanntmachung des Hinweises auf die Patenterteilung:
**02.01.92 Patentblatt 92/01**

(84) Benannte Vertragsstaaten:
**DE FR GB**

(56) Entgegenhaltungen:

**CHEMICAL ABSTRACTS, Band 83, Nr. 13, 29. September 1975, Seite 611, Nr. 114934b, Columbus, Ohio, US; & JP-A-75 46 656 (T. TADASHI et al.) 25-04-1975**

**CHEMICAL ABSTRACTS, Band 98, Nr. 3, 17. Januar 1983, Seite 546, Nr. 17055t, Columbus, Ohio, US; & JP-A-57 108 053 (MITSUBISHI CHEMICAL IND., CO., LTD) 05-07-1982**

**CHEMICAL ABSTRACTS, Band 87, Nr. 11, 12. September 1977, Seite 627, Nr. 85244b, Columbus, Ohio, US; && JP-A-77 25 768 (T. TAKEUCHI et al.) 25-02-1977**

(73) Patentinhaber: **HOECHST AKTIENGESELLSCHAFT
Postfach 80 03 20
W-6230 Frankfurt am Main 80(DE)**

(72) Erfinder: **Teetz, Volker, Dr.
An der Tann 20
W-6238 Hofheim am Taunus(DE)**
Erfinder: **Henke, Stephan, Dr.
Sophienruhe 4
W-6232 Bad Soden 2(DE)**
Erfinder: **Brocks, Dietrich, Dr.
Goethering 9
W-6200 Wiesbaden 42(DE)**
Erfinder: **Hanauske-Abel, Hartmut, Dr.
Schlossstrasse 35
W-6501 Dexheim(DE)**
Erfinder: **Günzler, Volkmar, Dr.
Marburger Strasse 2
W-3550 Marburg-Cappel(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Aus dem US-Patent 4 457 936 sind Hydroxyophenylthiazol-, -thiazolin- und -thiazolidin-carbonsäuren und ihre Verwendung als Inhibitoren der Prolin- und Lysinhydroxylase bekannt.

Es wurde gefunden, daß bestimmte Peptid-Derivate von α, ω-Diaminoalkancarbonsäuren mit hydroxysubstituierten Phenylacyl in der $N^{\omega}$ - Position hochwirksame Inhibitoren hydroxylierender Enzyme sind.

Die Erfindung betrifft daher Verbindungen der Formel I

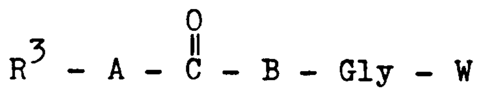

(I)

in welcher

$R^3$ = ($C_1$ - $C_8$)-Alkyl, das gegebenenfalls durch Carboxy, Amino, Hydroxy oder ($C_1$ - $C_4$)-Alkoxy monosubstituiert ist;
($C_3$-$C_8$)-Cycloalkyl;
($C_3$-$C_8$)-Cycloalkyl-($C_1$-$C_5$)-alkyl;
($C_6$-$C_{10}$)-Aryl oder ($C_6$-$C_{10}$)-Aryl-($C_1$-$C_5$)-alkyl,
die beide im Arylteil gegebenenfalls durch einen oder zwei gleiche oder verschiedene Reste aus der Reihe Carboxy, Amino, Hydroxy, ($C_1$-$C_4$)-Alkoxy oder Halogen substituiert sind;
($C_3$-$C_9$)-Heteroaryl oder
($C_3$-$C_6$)-Heteroaryl-($C_1$-$C_5$)-alkyl bedeutet;

A = für -O-, -NH- oder eine direkte Bindung steht;

B = einen Rest der Formel IIIa oder IIIb bedeutet;

(IIIa) 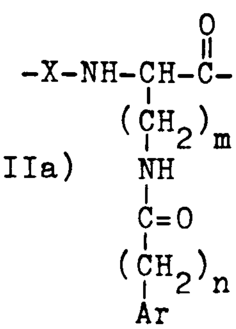

(IIIb) 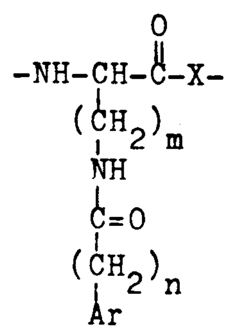

worin

X für einen Rest von Prolin oder 4-Thiaprolin oder einen Rest der Formel IV steht,

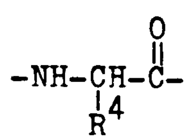

(IV)

in der $R^4$ Wasserstoff oder ($C_1$-$C_4$)-Alkyl, das gegebenenfalls durch Hydroxy, Carboxy, Carbamoyl, Methylthio, Phenyl, 4-Hydroxyphenyl, 4-Imidazolyl oder 3-Indolyl monosubstituiert ist, bedeutet;

m für 2, 3 oder 4;

n für 0 oder 1 und

Ar für Mono-, Di- oder Trihydroxyphenyl stehen und

W Hydroxy oder ein Rest der Formel V bedeutet,

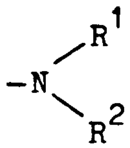

(V)

worin

- $R^1$ Wasserstoff, $(C_1-C_6)$-Alkyl oder $(C_3-C_6)$-Cycloalkyl und
- $R^2$ Wasserstoff, $(C_1-C_6)$-Alkyl oder $(C_3-C_6)$-Cycloalkyl bedeuten oder
- $R^1$ und $R^2$ zusammen für $-[CH_2]_4-$ oder $-[CH_2]_5-$ stehen, wobei eine $[CH_2]$-Gruppe durch -O- oder -S- ersetzt sein kann,

sowie deren physiologisch verträglichen Salze.

Alkyl kann geradkettig oder verzweigt sein. Cycloalkyl kann wie z.B. in 4-Methylcyclohexyl (eine) Alkylseitenkette(n) tragen.

Unter Aryl wird beispielsweise Phenyl oder Naphthyl, vorzugsweise aber Phenyl verstanden.

Ein Heteroaryl-Rest im Sinne der vorliegenden Erfindung ist der Rest eines monocyclischen oder bicyclischen $(C_3-C_9)$-Heteroaromaten, der im Ringsystem ein oder zwei N-Atome und/oder ein S- oder ein O-Atom enthält. Zum Begriff "Heteroaromat" siehe Garratt, Vollhardt, Aromatizität, Stuttgart 1973, Seiten 131 - 153. Beispiele geeigneter Heteroaryl-Reste sind die Reste von Thiophen, Furan, Benzothiophen, Benzofuran, Pyrrol, Imidazol, Pyrazol, Pyridin, Pyrazin, Pyrimidin, Pyridazin, Indol, Chinolin, Isochinolin, Oxazol, Isoxazol, Thiazol und Isothiazol.

Chiralitätszentren können, wenn nicht anders angegeben, sowohl in der R- als auch in der S-Konfiguration vorliegen.

- $R^3$ steht vorzugsweise für gegebenenfalls substituiertes $(C_1-C_8)$-Alkyl, Cyclopropyl, Cyclobutyl, Cyclopentyl, Cyclohexyl, Cyclopropylmethyl, Cyclobutylmethyl, Cyclopentylmethyl, Cyclohexylmethyl, gegebenenfalls substituiertes Phenyl, gegebenenfalls substituiertes Benzyl, gegebenenfalls substituiertes Phenethyl, einen Heteroaryl-Rest aus der Reihe 2-, 3- oder 4-Pyridyl, 2- oder 3-Pyrrolyl, 2- oder 3-Furyl, 2- oder 3-Thienyl, 4-Imidazolyl oder 1-, 3- oder 4-Isochinolyl oder einen solchen, über $-CH_2-$ oder $-CH_2-CH_2-$ gebundenen Heteroaryl-Rest, insbesondere für $(C_1-C_8)$-Alkyl (z.B. Methyl, Ethyl), durch Amino, Hydroxy, Carboxy, Methoxy oder Ethoxy monosubstituiertes $(C_1-C_8)$-Alkyl; Cyclopentyl, Cyclohexyl, Phenyl oder Mono- oder Dihalogenphenyl.
- B ist der Rest eines Dipeptids, das aus einer basischen R- oder S-konfigurierten Aminosäure wie $\alpha,\gamma$-Diaminobuttersäure, Ornithin oder Lysin besteht, welche entweder über die $\alpha$-Amino- oder die Carboxygruppe mit dem Rest X über eine Peptidbindung verknüpft ist, und deren $\omega$-Aminogruppe mit substituiertem Benzoyl oder Phenylacetyl durch Amidbindung verbunden ist und durch die oben genannten Teilformeln IIIa und IIIb wiedergegeben wird.
- X bedeutet vorzugsweise einen Rest von Prolin oder 4-Thiaprolin oder einen Rest der Formel IV , worin $R^4$ für Wasserstoff, Methyl, Hydroxymethyl, 1-Hydroxyethyl, Isopropyl, Isobutyl, sec. Butyl, Carboxymethyl, 2-Carboxyethyl, Carbamoylmethyl, 2-Carbamoylethyl, 2-Methylthioethyl, Phenylmethyl, (4-Hydroxyphenyl)-methyl, 4-Imidazolylmethyl oder 3-Indolylmethyl steht, und liegt vorzugsweise in der S-Konfiguration vor. Insbesondere bedeutet X den Rest des 4-Thiaprolins oder den Rest einer natürlich vorkommenden $\alpha$-Aminosäure (siehe Schröder, Lübke, The Peptides, Volume I, New York 1965, Seiten 137 - 270), wie Gly, Pro, His, Glu oder Leu.

Der Rest Ar trägt vorzugsweise in 2- und/oder in 3-Position eine Hydroxygruppe. Geeignete Reste Ar sind beispielsweise 2-Hydroxyphenyl, 3-Hydroxyphenyl, 4-Hydroxyphenyl, 2,3-Dihydroxyphenyl, 3,4-Dihydroxyphenyl oder 3,4,5-Trihydroxyphenyl.

Der C-terminale Rest W weist vorzugsweise die Bedeutungen Hydroxy, Amino, $(C_1-C_6)$-Alkylamino, Cyclohexylamino, Dimethylamino, Diethylamino, Ethylmethylamino, 1-Piperidyl oder 1-Pyrrolidyl auf.

Als Salze kommen insbesondere Alkali- und Erdalkalisalze, Salze mit physiologisch verträglichen Aminen und Salze mit anorganischen oder organischen Säuren wie beispielsweise Salzsäure, Bromwasserstoffsäure, Schwefelsäure, Maleinsäure oder Fumarsäure in Betracht.

Die Erfindung betrifft auch ein Verfahren zur Herstellung von Verbindungen der Formel I, das dadurch gekennzeichnet ist, daß man die Verbindungen in an sich bekannter Weise durch Kondensation der Fragmente aufbaut, gegebenenfalls temporär eingeführte Schutzgruppen abspaltet und die so erhaltenen Verbindungen der Formel I gegebenenfalls in ihre physiologisch verträglichen Salze überführt. Unter Fragmenten im obigen Sinne werden Aminosäuren oder deren Derivate sowie mehrere Aminosäuren enthaltende Segmente (wie z.B. $R^3$-A-CO-B-OH) verstanden.

Die Kondensation kann beispielsweise dadurch erfolgen, daß man ein Fragment einer Verbindung der

Formel I mit einer endständigen Carboxylgruppe oder einem reaktivem Säurederivat mit einem entsprechendem anderen Fragment, welches eine freie Aminogruppe enthält, wobei eventuell vorhandene weitere funktionelle Gruppen gegebenenfalls geschützt sein können, unter Bildung einer Amidbindung kondensiert. Methoden, die zur Herstellung eine Amidbindung geeignet sind werden in Houben-Weyl, Methoden der organischen Chemie, Band 15/2 beschrieben. Vorzugsweise werden die folgenden Methoden herangezogen: Aktivestermethode mit N-Hydroxysuccinimid als Esterkomponente, Kupplung mit DCC/HOBt und Kupplung mit Propanphosphonsäureanhydrid.

Zum temporären Schutz weiterer funktioneller Gruppen sind Schutzgruppen geeignet, die unter Bedingungen abspaltbar sind, unter denen die Reste Ar-$[CH_2]_n$-CO- und $R^3$-A-CO-am Molekül bleiben. Schutzgruppen in der Peptidsynthese sind beispielsweise beschrieben im Kontakte Merck 3/79, Seiten 14 - 22 und 1/80, Seiten 23 - 35.

Da die erfindungsgemäßen Verbindungen der Formel I enzymatisch katalysierte Hydroxylierungsreaktionen hemmen, sind sie geeignet, die Reifung von Proteinen zu verhindern, die erst in hydroxylierter Form funktionell aktiv sind und können daher als Fibrosuppressiva, Immunsuppressiva oder Cytostatika eingesetzt werden. Charakteristikum verschiedener Proteine wie z.B. der Kollagene oder C1q ist es, daß sie in funktionell aktiver Form Aminosäuren enthalten, die post-translational hydroxyliert werden. Wird diese Hydroxylierung durch Hemmung der diese Reaktion katalysierenden Hydroxylasen wie 4-Prolylhydroxylase, 3-Prolylhydroxylase, Lysylhydroxylase oder Desoxyhypusinhydroxylase unterbunden, so können diese Proteine ihre physiologische Funktion nicht mehr wahrnehmen.

Die Hemmwirkung der erfindungsgemäßen Substanzen beispielsweise auf die Kollagen-Biosynthese kann in einem Enzymtest analog der Methode von B. Peterkovsky und R. Di Blasio Anal. Biochem. 66, 279 - 286 (1975) getestet werden. Dabei wird unterhydroxyliertes Kollagen in Gegenwart von Eisen(II)-Ionen, α-Ketoglutarat und Ascorbat enzymatisch hydroxyliert. Als Enzyme können 4-Prolylhydroxylase, 3-Prolylhydroxylase oder Lysylhydroxylase in einem zellfreien Testmedium eingesetzt werden. Die Hemmwirkung kann auch in der Zell- oder Gewebekultur gemessen werden und wird als 50 %ige Hemmung der Enzym-Reaktion ($IC_{50}$) angegeben.

In Tabelle I sind die $IC_{50}$-Werte einiger der erfindungsgemäßen Verbindungen aufgeführt.

Tabelle I

| Verbindung | Prolylhydroxylase $IC_{50}$ [$10^{-6}$ mol/l] | Lysylhydroxylase $IC_{50}$[$10^{-6}$ mol/l] |
|---|---|---|
| Ac-Orn(2,3-Dihydroxybenzoyl)-Pro-Gly-OH | 38 | 7 |
| Ac-Orn(3,4-Dihydroxybenzoyl)-Pro-Gly-OH | 72 | 7 |
| Ac-Orn(3,4,5-Trihydroxybenzoyl)-Pro-Gly-OH | 29 | 8 |
| Ac-Orn(3,4-Dihydroxyphenylacetyl)-Pro-Gly-OH | 140 | 37 |
| Ac-Pro-Orn(3,4-Dihydroxybenzoyl)-Gly-OH | 50 | 39 |
| Ac-Pro-Orn(3,4,5-Trihydroxybenzoyl)-Gly-OH | 17 | 26 |
| Ac-Pro-Orn(3,4-Dihydroxyphenylacetyl)-Gly-OH | 155 | 70 |

Die Erfindung betrifft daher weiterhin die Verwendung von Verbindungen der Formel I zur Hemmung hydroxylierender Enzyme, Verbindungen der Formel I zur Anwendung als Fibrosuppresiva , Immunsuppresiva oder Cytostatika sowie pharmazeutische Mittel, die eine wirksame Menge dieser Verbindungen und einen physiologisch unbedenklichen Träger enthalten. Die Anwendung kann intranasal, intravenös, subcutan oder peroral erfolgen. Die Dosierung des Wirkstoffes hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsform gebracht, wie Tabletten, Dragees, Steckkapseln, wäßrige, alkoholische oder ölige Suspensionen oder wäßrige, alkoholische oder ölige Lösungen. Als inerte Träger kommen z.B. Gummi arabicum, Magnesiumcarbonat, Kaliumphosphat, Milchzucker, Glucose oder Stärke, insbesondere Maisstärke in Betracht. Dabei kann die Zubereitung sowohl als Trockenals auch als Feuchtgranulat erfolgen. Als ölige Trägerstoffe oder Lösungsmittel kommen beispielsweise pflanzliche oder tierische Öle in Betracht, wie Sonnenblumenöl oder Lebertran.

Zur subcutanen oder intravenösen Applikation werden die aktiven Verbindungen oder deren physiologisch verträglichen Salze, gewünschtenfalls mit den dafür üblichen Substanzen wie Lösungsmittler oder weiteren Hilfsstoffen in Lösung, Suspension oder Emulsion gebracht. Dazu kommen z.B. in Frage: Wasser, Propandiol oder Glycerin, daneben auch Zuckerlösungen wie Glucose- oder Mannitlösungen oder auch eine Mischung aus den genannten Lösungsmitteln.

Die folgenden Beispiele dienen zur Illustration der vorliegenden Erfindung, ohne daß diese darauf beschränkt wäre.

Verzeichnis der verwendeten Abkürzungen:

| | |
|---|---|
| AA | Aminosäureanalyse |
| Ac | Acetyl |
| Boc | tert.-Butoxycarbonyl |
| DC | Dünnschichtchromatographie |
| DCC | Dicyclohexylcarbodiimid |
| DCH | Dicyclohexylharnstoff |
| DMF | Dimethylformamid |
| DMSO | Dimethylsulfoxid |
| EE | Essigsäureethylester |
| FAB | Fast atom bombardment |
| Fmoc | 9-Fluorenylmethyloxycarbonyl |
| HOBt | 1-Hydroxybenzotriazol |
| M | Molekularpeak |
| MeOH | Methanol |
| MS | Massenspektrum |
| NEM | N-Ethylmorpholin |
| Schmp. | Schmelzpunkt |

Fortsetzung:

| | |
|---|---|
| THF | Tetrahydrofuran |
| Z | Benzyloxycarbonyl |

Die sonstigen für Aminosäuren und Schutzgruppen verwendeten Abkürzungen entsprechen dem in der Peptidchemie üblichen Buchstaben-Code, wie er z.B. in Europ. J. Biochem. 138, 9 - 37 (1984) beschrieben ist. Falls nicht ausdrücklich anders angegeben, handelt es sich immer um Aminosäuren der L-Konfiguration.

| Chromatographie-Laufmittelsysteme: | |
|---|---|
| 1) $CHCl_3$/MeOH | 9 : 1 |
| 2) $CHCl_3$/MeOH | 8 : 3 |
| 3) $CHCl_3$/MeOH/$CH_3COOH$/$H_2O$ | 20 : 15 : 2 : 2 |
| 4) $CHCl_3$/MeOH/$CH_3COOH$ | 50 : 10 : 5 |

Beispiel 1

Ac-Orn(2,3-Dihydroxybenzoyl)-Pro-Gly-OH

500 mg Ac-Orn-Pro-Gly-OBzl, 314 mg 2,3-Dibenzyloxybenzoesäure, 127 mg HOBt und 0,12 ml NEM werden in 20 ml DMF gelöst. Nach Zugabe von 194 mg DCC läßt man 18 Stunden bei Raumtemperatur reagieren. Das Lösungsmittel wird im Vakuum eingedampft, der Rückstand in EE gelöst und DCH abfiltriert. Einengen im Vakuum und Chromatographie des Rückstandes an Kieselgel (System 1) liefert 600 mg Ac-Orn (2,3-Dibenzyloxybenzoyl)-Pro-Gly-OBzl als farbloses Öl, das direkt der weiteren Reaktionsfolge unterworfen wird. Hierzu werden 600 mg des Produktes in 5 ml Methanol gelöst und nach Zugabe von Pd/C bei Raumtemperatur hydriert. Man filtriert nach 2 Stunden vom Katalysator ab, engt im Vakuum ein und chromatographiert an Kieselgel (System 3).

Es verbleiben 164 mg Ac-Orn(2,3-Dihydroxybenzoyl)-Pro-Gly-OH. $R_f$ (System 3) = 0,5; MS (FAB): 465 (M+1).

Das Ausgangsmaterial wird nach folgenden Arbeitsvorschriften hergestellt:

a) Ac-Orn-Pro-Gly-OBzl

2,0 g Ac-Orn(Boc)-Pro-Gly-OBzl werden in 5 ml Trifluoressigsäure gelöst und 1 Stunde bei Raumtemperatur gerührt. Nach Eindampfen im Hochvakuum verbleiben 2,1 g des Trifluoracetats von Ac-Orn-Pro-Gly-OBzl. $R_f$ (System 2) = 0,3; MS (FAB): 419 (M + 1).

b) Ac-Orn(Boc)-Pro-Gly-OBzl

Man löst 6,4 g H-Pro-Gly-OBzl, 4,6 g Ac-Orn(Boc)-OH, 3,0 ml NEM und 3,1 g HOBt in 30 ml EE und versetzt anschließend mit 4,8 g DCC in 10 ml EE. Nach 24 Stunden Rühren bei Raumtemperatur wird abfiltriert und eingedampft. Durch Chromatographie des Rückstands in Kieselgel (System 1) isoliert man 4,7 g (Ausbeute: 41%) der Zielverbindung als farbloses Pulver. $R_f$ (System 1) = 0,3; MS (FAB): 519 (M + 1).

c) H-Pro-Gly-OBzl

5,0 g Boc-Pro-OH, 7,4 g H-Gly-OBzl, 3,0 ml NEM und 3,1 g HOBt werden in 50 ml EE unter Zusatz von 5 ml DMF gelöst. Man versetzt mit 4,8 g DCC und läßt 48 Stunden bei Raumtemperatur rühren. Nach Einengen im Vakuum wird der Rückstand in 50 ml EE gelöst, 2 mal mit 30 ml wäßriger Citronensäurelösung und 2 mal mit ges. wäßriger Natriumhydrogencarbonatlösung ausgeschüttelt, die organische Phase über Natriumsulfat getrocknet, filtriert und eingedampft. Man löst den Rückstand in 10 ml Trifluoressigsäure, läßt 1 Stunde bei Raumtemperatur reagieren und engt im Vakuum ein. Der Rückstand kristallisiert aus EE/Pentan.
Man erhält 6,6 g (Ausbeute: 61%) H-Pro-Gly-OBzl•Trifluoracetat. MS (FAB): 263 (M + 1); $R_f$ (System 4) = 0,29.

d) Ac-Orn(Boc)-OH

9,3 g H-Orn(Boc)-OH, 5,0 g des N-Hydroxysuccinimidesters der Essigsäure und 4,1 ml NEM in 50 ml DMF und 10 ml $H_2O$ läßt man 3 Tage bei Raumtemperatur reagieren. Man dampft das Lösungsmittel im Vakuum ein und chromatographiert den Rückstand an Kieselgel (System 4). 9,6 g Ac-Orn(Boc)-OH (Ausbeute: 88%) kristallisieren leicht aus EE/Diethylether. MS (FAB): 275 (M + 1).

e) 2,3-Dibenzyloxybenzoesäure

19,8 g 2,3-Dibenzyloxybenzaldehyd werden in 100 ml Aceton gelöst. Unter kräftigem Rühren und Erwärmen auf 40 ° C gibt man eine Lösung aus 4,0 g $KMnO_4$ in 90 ml $H_2O$ innerhalb 45 min zu, wobei der ausgefallene Benzaldehyd unter Zusatz von insgesamt 150 ml Aceton wieder gelöst wird. Nach vollständiger Zugabe der $KMnO_4$-Lösung wird eine Stunde unter Rückfluß gekocht. Die heiße Lösung wird anschließend filtriert und der Niederschlag mit heißem Wasser gewaschen. Beim Ansäuren der kalten, wäßrigen Lösung mit verd. HCl kristallisiert die Carbonsäure in feinen weißen Nadeln aus. Man erhält 18,0 g der Zielverbindung (Ausbeute 92%). MS: 334 ($M^+$); Schmp.: 185 - 187 ° C.

f) 2,3-Dibenzyloxybenzaldehyd

13,8 g 2,3-Dihydroxybenzaldehyd, 30,4 g Benzylchlorid und 17,3 g pulverisiertes wasserfreies Kaliumcarbonat in 160 ml wasserfreiem Ethanol werden unter Feuchtigkeitsschluß 6 Stunden unter Rückfluß gekocht. Man filtriert die Reaktionslösung, wäscht mit Ethanol und dampft im Vakuum ein. Beim Digerieren des Rückstandes mit Diisopropylether kristallisiert die bekannte Zielverbindung in weißen Nadeln (Schmp. 89 - 91 ° C) aus.

Analog der in Beispiel 1 beschriebenen Verfahrensweise werden die Verbindungen der Beispiele 2 - 18 aus Ac-Orn-OH, Ac-Lys-OH oder Ac-Dab-OH durch Umsetzung mit H-Pro-Gly-OBzl oder H-His-Gly-OBzl und den entsprechenden Benzoesäure- oder Phenylessigsäurederivaten hergestellt.

| Beispiel Nr. | Verbindung |
|---|---|
| 2 | Ac-Orn(2-Hydroxybenzoyl)-Pro-Gly-OH |
| 3 | Ac-Orn(3-Hydroxybenzoyl)-Pro-Gly-OH |
| 4 | Ac-Orn(3,4-Dihydroxybenzoyl)-Pro-Gly-OH |
| 5 | Ac-Orn(3,4,5-Trihydroxybenzoyl)-Pro-Gly-OH |
| 6 | Ac-Orn(2,3-Dihydroxyphenylacetyl)-Pro-Gly-OH |
| 7 | Ac-Orn(3,4-Dihydroxyphenylacetyl)-Pro-Gly-OH |
| 8 | Ac-Orn(2,3-Dihydroxybenzoyl)-His-Gly-OH |
| 9 | Ac-Orn(3,4-Dihydroxybenzoyl)-His-Gly-OH |
| 10 | Ac-Orn(3,4-Dihydroxyphenylacetyl)-His-Gly-OH |
| 11 | Ac-Lys(3,4-Dihydroxybenzoyl)-Pro-Gly-OH |
| 12 | Ac-Lys(3,4-Dihydroxyphenylacetyl)-Pro-Gly-OH |
| 13 | Ac-Lys(3,4-Dihydroxybenzoyl)-His-Gly-OH |
| 14 | Ac-Lys(3,4-Dihydroxyphenylacetyl)-His-Gly-OH |
| 15 | Ac-Dab(3,4-Dihydroxybenzoyl)-Pro-Gly-OH |
| 16 | Ac-Dab(3,4-Dihydroxyphenylacetyl)-Pro-Gly-OH |
| 17 | Ac-Dab(3,4-Dihydroxybenzoyl)-His-Gly-OH |
| 18 | Ac-Dab(3,4-Dihydroxyphenylacetyl)-His-Gly-OH |

Um die Strukturen der so hergestellten Peptide zu bestätigen, wurden verschiedenartige analytische und spektroskopische Methoden angewandt.
Einige Ergebnisse sind in Tabelle 2 zusammengefaßt.

Tabelle 2

| Beispiel Nr. | MS(FAB) | DC($R_f$) | $^1$H-NMR a) | Sonst. |
|---|---|---|---|---|
| 2 | 449 (M + 1) | 0,60 (Syst.2) | + | |
| 3 | 449 (M + 1) | 0,58 (Syst.2) | + | AA |
| 4 | 465 (M + 1) | 0,48 (Syst.2) | + | |
| 5 | 481 (M + 1) | 0,50 (Syst.3) | + | |
| 6 | 479 (M + 1) | 0,55 (Syst.2) | + | |
| 7 | 479 (M + 1) | 0,69 (Syst.3) | + | |
| 8 | 505 (M + 1) | 0,26 (Syst.2) | + | AA |
| 9 | 505 (M + 1) | 0,45 (Syst.2) | + | C,H,N-Analyse |
| 10 | 519 (M + 1) | 0,35 (Syst.2) | + | C,H,N-Analyse |
| 11 | 479 (M + 1) | 0,50 (Syst.2) | + | |
| 12 | 493 (M + 1) | 0,50 (Syst.2) | + | |
| 13 | 519 (M + 1) | 0,32 (Syst.2) | + | AA |
| 14 | 533 (M + 1) | 0,35 (Syst.2) | + | |
| 15 | 451 (M + 1) | 0,38 (Syst.2) | + | |
| 16 | 465 (M + 1) | 0,40 (Syst.2) | + | |
| 17 | 491 (M + 1) | 0,18 (Syst.2) | + | |
| 18 | 505 (M + 1) | 0,25 (Syst.2) | + | |

a) gemessen bei 60 bzw. 270 MHz;
" + " bedeutet: in Einklang mit der angegebenen Struktur.

Beispiel 19

Ac-Pro-Orn(3,4-Dihydroxybenzoyl)-Gly-OH

0,33 g 3,4-Dibenzyloxybenzoesäure, 0,14 g HOBt und 0,45 g Ac-Pro-Orn-Gly-OBzl werden in 5 ml DMF gelöst. Anschließend kühlt man auf -10 °C und versetzt mit 0,23 g DCC in 1 ml DMF und 0,13 ml NEM. Man hält das Reaktionsgemisch 1 Stunde bei -10 °C und rührt dann 24 Stunden bei Raumtemperatur. Nach dem Abfiltrieren des Dicyclohexylharnstoffs wird im Vakuum eingedampft und der Rückstand an Kieselgel

chromatographiert (System 1). Man erhält 0,24 g (Ausbeute: 31%) Ac-Pro-Orn(3,4-Dibenzyloxybenzoyl)-Gly-OBzl als amorphen Feststoff, der direkt weiterverarbeitet wird. Hierzu löst man das Produkt in 10 ml Methanol und versetzt mit wenig Pd/C. Es wird bis zur vollständigen hydrogenolytischen Spaltung bei Raumtemperatur $H_2$ eingeleitet (DC-Kontrolle), dann filtriert und das Lösungsmittel im Vakuum eingeengt. Es verbleiben 150 mg der Zielverbindung. $R_f$ (System 3) = 0,36; MS (FAB): 465 (M+1).

Das Ausgangsmaterial wird nach folgenden Arbeitsvorschriften hergestellt:

a) Ac-Pro-Orn-Gly-OBzl

1,0 g Ac-Pro-Orn(Boc)-Gly-OBzl werden in 5 ml Trifluoressigsäure gelöst und 1 Stunde bei Raumtemperatur gerührt. Nach Eindampfen im Hochvakuum verbleiben 1,0 g des Trifluoracetats von Ac-Pro-Orn-Gly-OBzl. $R_f$ (System 2) = 0,6; MS (FAB): 419 (M+1).

b) Ac-Pro-Orn(Boc)-Gly-OBzl

5,6 g Ac-Pro-Orn(Boc)-OH, 5,0 g des Tosylats von H-Gly-OBzl und 2,0 g HOBt werden in 50 ml DMF gelöst, auf 0 °C gekühlt und mit 1,9 ml NEM und 3,4 g DCC in 5 ml DMF versetzt. Man rührt eine Stunde bei 0 °C und über Nacht bei Raumtemperatur. Nach dem Abfiltrieren des Harnstoffs wird das Lösungsmittel im Vakuum eingedampft und der Rückstand an Kieselgel chromatographiert (System 4). Es verbleiben 6,1 g (Ausbeute: 79%) Ac-Pro-Orn(Boc)-Gly-OBzl als Öl. $R_f$ (System 4) = 0,75; MS (FAB): 519 (M+1).

c) Ac-Pro-Orn(Boc)-OH

5,5 g H-Orn(Boc)-OH und 6,0 g Ac-Pro-OSU werden in 50 ml DMF gelöst und nach Zugabe von 3 ml NEM 24 Stunden bei Raumtemperatur gerührt. Nach dem Abdestillieren des Lösungsmittels in Vakuum nimmt man den Rückstand in 30 ml n-Butanol auf, wäscht 2 mal mit ges. wäßriger Kaliumhydrogensulfatlösung und anschließend mit ges. wäßriger Natriumchloridlösung. Man engt im Vakuum ein und fällt Ac-Pro-Orn(Boc)-OH durch Digerieren mit Diethylether. $R_f$ (System 3) = 0,24; MS (FAB): 372 (M+1)

d) Ac-Pro-OSU

Die Lösung von 39,3 g Ac-Pro-OH und 28,7 g N-Hydroxysuccinimid in 200 ml Dioxan wird auf -5 °C gekühlt und mit einer vorgekühlten Lösung von 51,6 g DCC in 100 ml Dioxan versetzt. Man rührt 1 Stunde bei 0 °C, eine weitere Stunde bei Raumtemperatur und filtriert dann vom ausgefallenen DCH ab, der gründlich mit 2 mal 50 ml Dioxan gewaschen wird. Nach dem Abziehen des Lösungsmittels im Vakuum wird der Rückstand in 30 ml Isopropanol aufgenommen und das Produkt durch Zusatz von Petrolether über Nacht ausgefällt. Man erhält 33,0 g (Ausbeute: 50%) fein kristallines Ac-Pro-OSU.
MS (FAB): 255 (M+1); Schmp. 100 - 105 °C.

Analog der in Beispiel 19 beschriebenen Verfahrensweise werden die Verbindungen der Beispiele 20 - 43 aus Ac-Pro-OH, Ac-Gly-OH, Ac-Glu-OH oder Ac-His-OH durch Umsetzung mit Ornithin, $\alpha,\gamma$-Diaminobuttersäure oder Lysin, sowie Glycin und den entsprechenden Benzoesäure- oder Phenylessigsäurederivaten dargestellt.

| Beispiel Nr. | Verbindung |
|---|---|
| 20 | Ac-Pro-Orn(2-Hydroxybenzoyl)-Gly-OH |
| 21 | Ac-Pro-Orn(3-Hydroxybenzoyl)-Gly-OH |
| 22 | Ac-Pro-Orn(2,3-Dihydroxybenzoyl)-Gly-OH |
| 23 | Ac-Pro-Orn(3,4,5-Trihydroxybenzoyl)-Gly-OH |
| 24 | Ac-Pro-Orn(2,3-Dihydroxyphenylacetyl)-Gly-OH |
| 25 | Ac-Pro-Orn(3,4-Dihydroxyphenylacetyl)-Gly-OH |
| 26 | Ac-Pro-Dab(3,4-Dihydroxybenzoyl)-Gly-OH |
| 27 | Ac-Pro-Dab(3,4-Dihydroxyphenylacetyl)-Gly-OH |
| 28 | Ac-Pro-Lys(3,4-Dihydroxybenzoyl)-Gly-OH |
| 29 | Ac-Pro-Lys(3,4-Dihydroxyphenylacetyl)-Gly-OH |
| 30 | Ac-Gly-Orn(3,4-Dihydroxybenzoyl)-Gly-OH |
| 31 | Ac-Gly-Orn(3,4-Dihydroxyphenylacetyl)-Gly-OH |
| 32 | Ac-Gly-Dab(3,4-Dihydroxybenzoyl)-Gly-OH |
| 33 | Ac-Gly-Dab(3,4-Dihydroxyphenylacetyl)-Gly-OH |
| 34 | Ac-Gly-Lys(3,4-Dihydroxybenzoyl)-Gly-OH |
| 35 | Ac-Gly-Lys(3,4-Dihydroxyphenylacetyl)-Gly-OH |
| 36 | Ac-Glu-Orn(3,4-Dihydroxybenzoyl)-Gly-OH |
| 37 | Ac-Glu-Orn(3,4-Dihydroxyphenylacetyl)-Gly-OH |
| 38 | Ac-His-Orn(3,4-Dihydroxybenzoyl)-Gly-OH |
| 39 | Ac-His-Orn(3,4-Dihydroxyphenylacetyl)-Gly-OH |
| 40 | Ac-His-Dab(3,4-Dihydroxybenzoyl)-Gly-OH |
| 41 | Ac-His-Dab(3,4-Dihydroxyphenylacetyl)-Gly-OH |
| 42 | Ac-His-Lys(3,4-Dihydroxybenzoyl)-Gly-OH |
| 43 | Ac-His-Lys(3,4-Dihydroxyphenylacetyl)-Gly-OH |

Zur Bestätigung der Struktur der so hergestellten Peptide wurden verschiedenartige analytische und spektroskopische Methoden angewandt. Einige Ergebnisse sind in Tabelle 3 zusammengefaßt.

Tabelle 3

| Beispiel Nr. | MS(FAB) | DC($R_f$) | $^1$H-NMR a) | Sonst. |
|---|---|---|---|---|
| 20 | 449 (M+1) | 0,65 (Syst.2) | + | AA |
| 21 | 449 (M+1) | 0,48 (Syst.2) | + | AA |
| 22 | 465 (M+1) | 0,45 (Syst.3) | + | |
| 23 | 481 (M+1) | 0,31 (Syst.3) | + | |
| 24 | 479 (M+1) | 0,40 (Syst.3) | + | |
| 25 | 479 (M+1) | 0,42 (Syst.3) | + | |
| 26 | 451 (M+1) | 0,21 (Syst.3) | + | |
| 27 | 465 (M+1) | 0,30 (Syst.3) | + | AA |
| 28 | 479 (M+1) | 0,24 (Syst.2) | + | |
| 29 | 493 (M+1) | 0,25 (Syst.2) | + | C,H,N-Analyse |
| 30 | 425 (M+1) | 0,40 (Syst.3) | + | |
| 31 | 439 (M+1) | 0,38 (Syst.3) | + | |
| 32 | 411 (M+1) | 0,28 (Syst.3) | + | |
| 33 | 425 (M+1) | 0,30 (Syst.3) | + | |
| 34 | 439 (M+1) | 0,15 (Syst.4) | + | |
| 35 | 453 (M+1) | 0,25 (Syst.4) | + | |
| 36 | 497 (M+1) | 0,19 (Syst.3) | + | C,H,N-Analyse |
| 37 | 511 (M+1) | 0,25 (Syst.3) | + | |
| 38 | 505 (M+1) | 0,20 (Syst.2) | + | |
| 39 | 519 (M+1) | 0,24 (Syst.2) | + | |
| 40 | 491 (M+1) | 0,16 (Syst.2) | + | |
| 41 | 505 (M+1) | 0,16 (Syst.2) | + | |
| 42 | 519 (M+1) | 0,30 (Syst.2) | + | |
| 43 | 533 (M+1) | 0,31 (Syst.2) | + | |

a) gemessen bei 60 bzw. 270 MHz;
"+" bedeutet: in Einklang mit der angegebenen Struktur

**Patentansprüche**

**1.** Verbindung der Formel I

$$R^3 - A - \overset{\overset{\displaystyle O}{\|}}{C} - B - Gly - W \qquad (I)$$

in welcher

R³ = ($C_1$ - $C_8$)-Alkyl, das gegebenenfalls durch Carboxy, Amino, Hydroxy oder ($C_1$ - $C_4$)-Alkoxy monosubstituiert ist;
($C_3$-$C_8$)-Cycloalkyl;
($C_3$-$C_8$)-Cycloalkyl-($C_1$-$C_5$)-alkyl;
($C_6$-$C_{10}$)-Aryl oder ($C_6$-$C_{10}$)-Aryl-($C_1$-$C_5$)-alkyl,
die beide im Arylteil gegebenenfalls durch einen oder zwei gleiche oder verschiedene Reste aus der Reihe Carboxy, Amino, Hydroxy, ($C_1$-$C_4$)-Alkoxy oder Halogen substituiert sind;
($C_3$-$C_9$)-Heteroaryl oder
($C_3$-$C_6$)-Heteroaryl-($C_1$-$C_5$)-alkyl bedeutet;

A = für -O-, -NH- oder eine direkte Bindung steht;

B = einen Rest der Formel IIIa oder IIIb bedeutet;

(IIIa) 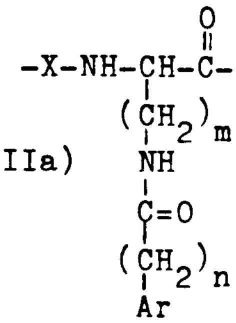 (IIIb) 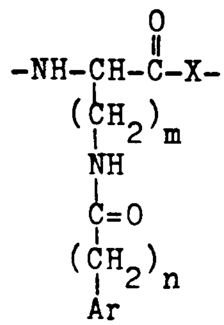

worin

X für einen Rest von Prolin oder 4-Thiaprolin oder einen Rest der Formel IV steht,

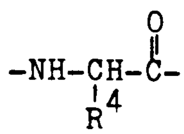 (IV)

in der $R^4$ Wasserstoff oder $(C_1\text{-}C_4)$-Alkyl, das gegebenenfalls durch Hydroxy, Carboxy, Carbamoyl, Methylthio, Phenyl, 4-Hydroxyphenyl, 4-Imidazolyl oder 3-Indolyl monosubstituiert ist, bedeutet;

m für 2, 3 oder 4;

n für 0 oder 1 und

Ar für Mono-, Di- oder Trihydroxyphenyl stehen und

W Hydroxy oder ein Rest der Formel V bedeutet,

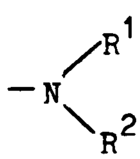 (V)

worin

$R^1$ Wasserstoff, $(C_1\text{-}C_6)$-Alkyl oder $(C_3\text{-}C_6)$-Cycloalkyl und

$R^2$ Wasserstoff, $(C_1\text{-}C_6)$-Alkyl oder $(C_3\text{-}C_6)$-Cycloalkyl bedeuten oder

$R^1$ und $R^2$ zusammen für $-[CH_2]_4-$ oder $-[CH_2]_5-$ stehen, wobei eine $[CH_2]$-Gruppe durch -O- oder -S- ersetzt sein kann,

sowie deren physiologisch verträglichen Salze.

**2.** Verbindung gemäß Anspruch 1, in welcher X einen Rest von Prolin oder 4-Thiaprolin oder einen Rest der Formel IV bedeutet, worin $R^4$ für Wasserstoff, Methyl, Hydroxymethyl, 1-Hydroxyethyl, Isopropyl, Isobutyl, sec. Butyl, Carboxymethyl, 2-Carboxyethyl, Carbamoylmethyl, 2-Carbamoylethyl, 2-Methylthioethyl, Phenylmethyl, (4-Hydroxyphenyl)-methyl, 4-Imidazolylmethyl oder 3-Indolylmethyl steht.

**3.** Verbindung gemäß Anspruch 1 oder 2, in welcher X für Gly, Pro, 4-Thia-Pro, His, Glu oder Leu steht.

**4.** Verbindung gemäß einem der Ansprüche 1 bis 3, in welcher $R^3$ für gegebenenfalls substituiertes $(C_1\text{-}C_8)$-Alkyl, Cyclopropyl, Cyclobutyl, Cyclopentyl, Cyclohexyl, Cyclopropylmethyl, Cyclobutylmethyl, Cyclopentylmethyl, Cyclohexylmethyl, gegebenenfalls substituiertes Phenyl, gegebenenfalls substituiertes Benzyl, gegebenenfalls substituiertes Phenethyl, einen Heteroaryl-Rest aus der Reihe 2-, 3- oder 4-Pyridyl, 2- oder 3-Pyrrolyl, 2- oder 3-Furyl, 2- oder 3-Thienyl, 4-Imidazolyl oder 1-, 3-oder 4-Isochinloyl oder einen solchen, über $-CH_2-$ oder $-CH_2\text{-}CH_2-$ gebundenen Heteroaryl-Rest steht.

**5.** Verbindung gemäß einem der Ansprüche 1 bis 4, in welcher $R^3$ für $(C_1-C_8)$-Alkyl, durch Amino, Hydroxy, Carboxy, Methoxy oder Ethoxy monosubstituiertes $(C_1-C_8)$-Alkyl, Cyclopentyl, Cyclohexyl, Phenyl oder Mono- oder Dihalogenphenyl steht.

**6.** Verbindung gemäß einem der Ansprüche 1 bis 5, in welcher in Position 2 und/oder 3 des Restes Ar eine Hydroxyl-Gruppe steht.

**7.** Verbindung gemäß einem der Ansprüche 1 bis 5, in welcher Ar für 2-Hydroxyphenyl, 3-Hydroxyphenyl, 4-Hydroxyphenyl, 2,3-Dihydroxyphenyl, 3,4-Dihydroxyphenyl oder 3,4,5-Trihydroxyphenyl steht.

**8.** Verbindung gemäß einem der Ansprüche 1 bis 7, in welcher W für Hydroxy, Amino, $(C_1-C_6)$-Alkylamino, Cyclohexylamino, Dimethylamino, Diethylamino, Ethylmethylamino, 1-Piperidyl oder 1-Pyrrolidyl steht.

**9.** Verfahren zur Herstellung einer Verbindung der Formel I gemäß einem der Ansprüche 1 - 8, dadurch gekennzeichnet, daß man die Verbindung durch Fragmentkondensation aufbaut, gegebenenfalls temporär eingeführte Schutzgruppen abspaltet und die so erhaltene Verbindung der Formel I gegebenenfalls in ihr physiologisch verträgliches Salz überführt.

**10.** Pharmazeutische Zubereitung enthaltend eine Verbindung gemäß einem der Ansprüche 1 - 8 und einem physiologisch unbedenklichen Träger.

**11.** Verwendung einer Verbindung gemäß einem der Ansprüche 1 - 8 zur Hemmung hydroxylierender Enzyme.

**12.** Verbindung gemäß einem der Ansprüche 1 - 8 zur Anwendung als Heilmittel.

**13.** Verbindung gemäß einem der Ansprüche 1 - 8 zur Anwendung als Fibrosupptessiva, Immunsuppressiva und/oder Cytostatika.

## Claims

**1.** A compound of the formula I

$$R^3 - A - \overset{\overset{\displaystyle O}{\|}}{C} - B - Gly - W \qquad (I)$$

in which

- $R^3$ denotes $(C_1-C_8)$-alkyl which is optionally monosubstituted by carboxyl, amino, hydroxyl or $(C_1-C_4)$-alkoxy; $(C_3-C_8)$-cycloalkyl;
  $(C_3-C_8)$-cycloalkyl-$(C_1-C_5)$-alkyl;
  $(C_6-C_{10})$-aryl or $(C_6-C_{10})$-aryl-$(C_1-C_5)$-alkyl,
  both of which can optionally be substituted in the aryl moiety by one or two, identical or different, radicals from the series comprising carboxyl, amino, hydroxyl, $(C_1-C_4)$-alkoxy or halogen;
  $(C_3-C_9)$-heteroaryl or
  $(C_3-C_6)$-heteroaryl-$(C_1-C_5)$-alkyl;
- A represents -O-, -NH- or a direct bond;
- B denotes a radical of the formula IIIa or IIIb;

(IIIa) 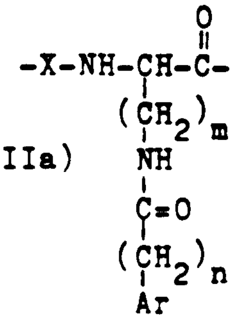

(IIIb) 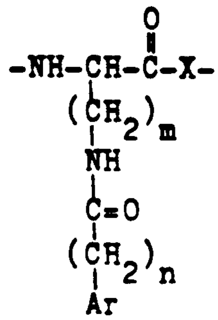

in which

X represents a radical of proline or 4-thiaproline or a radical of the formula IV

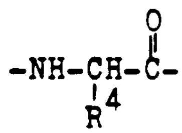 (IV)

in which

$R^4$ denotes hydrogen or ($C_1$-$C_4$)-alkyl which is optionally monosubstituted by hydroxyl, carboxyl, carbamoyl, methylthio, phenyl, 4-hydroxyphenyl, 4-imidazolyl or 3-indolyl;

m represents 2, 3 or 4,

n represents 0 or 1, and

Ar represents mono-, di- or trihydroxyphenyl, and

W denotes hydroxyl or a radical of the formula V

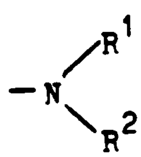 (V)

in which

$R^1$ denotes hydrogen, ($C_1$-$C_6$)-alkyl or ($C_3$-$C_6$)-cycloalkyl, and

$R^2$ denotes hydrogen, ($C_1$-$C_6$)-alkyl or ($C_3$-$C_6$)-cycloalkyl, or

$R^1$ and $R^2$ together represent -$[CH_2]_4$- or -$[CH_2]_5$-, it being possible for one $[CH_2]$ group to be replaced by -O- or -S-,

and its physiologically tolerated salts.

**2.** A compound as claimed in claim 1, in which X denotes a radical of proline or 4-thiaproline or a radical of the formula IV in which $R^4$ represents hydrogen, methyl, hydroxymethyl, 1-hydroxyethyl, isopropyl, isobutyl, sec.-butyl, carboxymethyl, 2-carboxyethyl, carbamoylmethyl, 2-carbamoylethyl, 2-methylthioethyl, phenylmethyl, (4-hydroxyphenyl)-methyl, 4-imidazolylmethyl or 3-indolylmethyl.

**3.** A compound as claimed in claim 1 or 2, in which X represents Gly, Pro, 4-Thia-Pro, His, Glu or Leu.

**4.** A compound as claimed in one of claims 1 to 3, in which $R^3$ represents optionally substituted ($C_1$-$C_8$)-alkyl, cyclopropyl, cyclobutyl, cyclopentyl, cyclohexyl, cyclopropylmethyl, cyclobutylmethyl, cyclopentylmethyl, cyclohexylmethyl, optionally substituted phenyl, optionally substituted benzyl, optionally substituted phenethyl, a heteroaryl radical from the series comprising 2-, 3- or 4-pyridyl, 2- or 3-pyrrolyl, 2- or 3-furyl, 2- or 3-thienyl, 4-imidazolyl or 1-, 3- or 4-isoquinolyl or such a heteroaryl radical which is bonded via -$CH_2$- or -$CH_2$-$CH_2$-.

**5.** A compound as claimed in one of claims 1 to 4, in which $R^3$ represents ($C_1$-$C_8$)-alkyl, ($C_1$-$C_8$)-alkyl which is monosubstituted by amino, hydroxyl, carboxyl, methoxy or ethoxy, or represents cyclopentyl,

cyclohexyl, phenyl or mono- or dihalogenophenyl.

**6.** A compound as claimed in one of claims 1 to 5, in which a hydroxyl group is present in position 2 and/or 3 of the radical Ar.

**7.** A compound as claimed in one of claims 1 to 5, in which Ar represents 2-hydroxyphenyl, 3-hydroxyphenyl, 4-hydroxyphenyl, 2,3-dihydroxyphenyl, 3,4-dihydroxyphenyl or 3,4,5-trihydroxyphenyl.

**8.** A compound as claimed in one of claims 1 to 7, in which W represents hydroxyl, amino, ($C_1$-$C_6$)-alkylamino,cyclohexylamino, dimethylamino, diethylamino, ethylmethylamino, 1-piperidyl or 1-pyrrolidyl.

**9.** A process for the preparation of a compound of the formula I as claimed in one of claims 1 - 8, which comprises synthesis of the compound by fragment condensation, where appropriate elimination of protective groups which have been temporarily introduced, and conversion of the resulting compound of the formula I where appropriate into its physiologically tolerated salt.

**10.** A pharmaceutical formulation containing a compound as claimed in one of claims 1-8 and a physiologically acceptable vehicle.

**11.** The use of a compound as claimed in one of claims 1-8 for the inhibition of hydroxylating enzymes.

**12.** A compound as claimed in one of claims 1-8 for use as medicine.

**13.** A compound as claimed in one of claims 1-8 for use as fibrosuppressants, immunosuppressants and/or cytostatics.

## Revendications

**1.** Composé de formule I

$$R^3\text{-}A\text{-}\overset{\overset{\displaystyle O}{\|}}{C}\text{-}B\text{-}Gly\text{-}W \qquad (I)$$

dans laquelle

- $R^3$ représente un radical alkyle en $C_1$-$C_8$ qui est éventuellement monosubstitué par un groupe carboxy, amino, hydroxy ou alcoxy en $C_1$-$C_4$;
  un radical cycloalkyle en $C_3$-$C_8$;
  un radical cycloalkyl($C_3$-$C_8$)-alkyle($C_1$-$C_5$);
  un radical aryle en $C_6$-$C_{10}$ ou aryl($C_6$-$C_{10}$)-alkyle($C_1$-$C_5$), qui sont l'un et l'autre éventuellement substitués dans le fragment aryle par un ou deux substituants, identiques ou différents, choisis parmi des atomes d'halogène et des groupes carboxy, amino, hydroxy et alcoxy en $C_1$-$C_4$;
  un radical hétéroaryle en $C_3$-$C_9$ ou
  un radical hétéroaryl($C_3$-$C_6$)-alkyle($C_1$-$C_5$);
- A représente -O-, -NH- ou une liaison directe;
- B représente un radical de formule IIIa ou IIIb;

(IIIa) 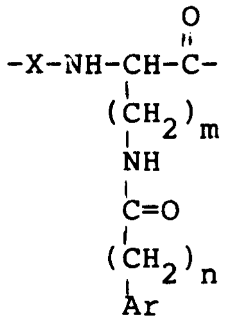 (IIIb) 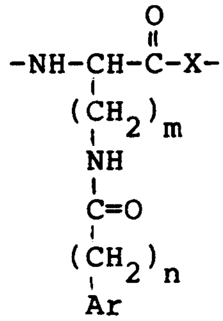

dans lesquelles

X représente un reste de proline ou 4-thiaproline, ou un radical de formule IV

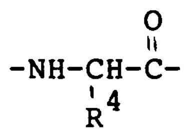 (IV)

dans laquelle

$R^4$ représente un atome d'hydrogène ou un groupe alkyle en $C_1$-$C_4$, qui est éventuellement monosubstitué par un groupe hydroxy, carboxy, carbamoyle, méthylthio, phényle, 4-hydroxyphényle, 4-imidazolyle ou 3-indolyle;

m est 2, 3 ou 4;

n est 0 ou 1; et

Ar représente un radical mono-, di- ou trihydroxyphényle et

W représente le groupe hydroxy ou un radical de formule V

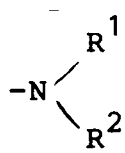 (V)

dans laquelle

$R^1$ représente un atome d'hydrogène ou un groupe alkyle en $C_1$-$C_6$ ou cycloalkyle en $C_3$-$C_6$, et

$R^2$ représente un atome d'hydrogène ou un groupe alkyle en $C_1$-$C_6$ ou cycloalkyle en $C_1$-$C_6$, ou

$R^1$ et $R^2$ représentent ensemble $-(CH_2)_4-$ ou $-(CH_2)_5-$, un groupe $(CH_2)$ pouvant être remplacé par -O- ou -S-,

et sels physiologiquement acceptables de celui-ci.

**2.** Composé selon la revendication 1, dans lequel X représente un reste de proline ou de 4-thiaproline ou un reste de formule IV, dans lequel $R^4$ représente un atome d'hydrogène ou le groupe méthyle, hydroxyméthyle, 1-hydroxyéthyle, isopropyle, isobutyle, sec-butyle, carboxyméthyle, 2-carboxyéthyle, carbamoylméthyle, 2-carbamoyléthyle, 2-méthylthioéthyle, phénylméthyle, (4-hydroxyphényl)-méthyle, 4-imidazolylméthyle ou 3-indolylméthyle.

**3.** Composé selon la revendication 1 ou 2, dans lequel X représente Gly, Pro, 4-Thia-Pro, His, Glu ou Leu.

**4.** Composé selon l'une des revendications 1 à 3, dans lequel $R^3$ représente un radical alkyle en $C_1$-$C_8$ éventuellement substitué, le radical cyclopropyle, cyclobutyle, cyclopentyle, cyclohexyle, cyclopropylméthyle, cyclobutylméthyle, cyclopentylméthyle, cyclohexylméthyle, un radical phényle éventuellement substitué, un radical benzyle éventuellement substitué, un radical phénéthyle éventuellement substitué, un radical hétéroaryle choisi parmi les radicaux 2-, 3- ou 4-pyridyle, 2- ou 3-pyrrolyle, 2- ou 3-furyle, 2-

ou 3-thiényle, 4-imidazolyle et 1-, 3- ou 4-isoquinolyle ou un tel radical hétéroaryle lié par l'intermédiaire de $-CH_2-$ ou $-CH_2-CH_2-$

**5.** Composé selon l'une des revendications 1 à 4, dans lequel $R^3$ représente un radical alkyle en $C_1$-$C_8$, un radical alkyle en $C_1$-$C_8$ monosubstitué par un groupe amino, hydroxy, carboxy, méthoxy ou éthoxy, un radical cyclopentyle, cyclohexyle, phényle ou mono- ou dihalogénophényle.

**6.** Composé selon l'une des revendications 1 à 5, dans lequel un groupe hydroxy se trouve en position 2 et/ou en position 3 du radical Ar.

**7.** Composé selon l'une des revendications 1 à 5, dans lequel Ar représente le groupe 2-hydroxyphényle, 3-hydroxyphényle, 4-hydroxyphényle, 2,3-dihydroxyphényle, 3,4-dihydroxyphényle ou 3,4,5-trihydroxyphényle.

**8.** Composé selon l'une des revendications 1 à 7, dans lequel W représente un radical hydroxy, amino, alkyl($C_1$-$C_6$)-amino, cyclohexylamino, diméthylamino, diéthylamino, éthylméthylamino, 1-pipéridyle ou 1-pirrolidyle.

**9.** Procédé pour la préparation d'un composé de formule I selon l'une des revendications 1 à 8, caractérisé en ce que l'on fait la synthèse du composé par condensation de fragments, éventuellement on élimine des groupes protecteurs introduits temporairement et éventuellement on convertit en l'un de ses sels physiologiquement acceptables le composé de formule I ainsi obtenu.

**10.** Composition pharmaceutique contenant un composé selon l'une des revendications 1 à 8 et un véhicule physiologiquement acceptable.

**11.** Utilisation d'un composé selon l'une des revendications 1 à 8, pour l'inhibition d'enzymes d'hydroxylation.

**12.** Composé selon l'une des revendications 1 à 8, pour utilisation en tant que médicament.

**13.** Composé selon l'une des revendications 1 à 8, pour utilisation en tant que fibrosuppresseur, immunosuppresseur et/ou cytostatique.